# EUROPEAN PATENT SPECIFICATION

(11) **EP 2 023 845 B1**
(45) Date of publication and mention of the grant of the patent: **07.07.2010**
(21) Application number: 07795082.2
(22) Date of filing: 17.05.2007
(51) Int. Cl.: A61B 19/00

(54) **Robotic instrument system**
Roboterinstrumentensystem
Système d'instrument robotisé

(30) Priority: 17.05.2006 US 801355 P; 17.05.2006 US 801546 P; 18.05.2006 US 801945 P
(43) Date of publication of application: 18.02.2009
(60) Divisional application: 10150323.3
(73) Proprietor: Hansen Medical, Inc., Mountain View, CA 94043 (US)
(72) Inventor: BARBAGLI, Federico, San Francisco, California 94131 (US)
(74) Representative: Lecomte, Didier
(86) International application number: PCT/US2007/012015
(87) International publication number: WO 2007/136803

(56) References cited:
- WO-A-2005/039835
- US-A- 5 762 458
- US-A1- 2006 084 945
- US-B1- 6 184 868
- DAVIES B L ET AL: "ACTIVE COMPLIANCE IN ROBOTIC SURGERY-THE USE OF FORCE CONTROL AS A DYNAMIC CONSTRAINT" PROCEEDINGS OF THE INSTITUTION OF MECHANICAL ENGINEERS. JOURNAL OF ENGINEERING IN MEDICINE. PART H, MECHANICAL ENGINEERING PUBLICATIONS LTD, LONDON, GB, vol. 211, no. PART H, 1997, pages 285-292, XP000730143 ISSN: 0954-4119

## Description

### FIEND OF INVENTION

The invention relates generally to robotically-controlled medical instrument systems, and more particularly to robotically-controlled flexible instrument systems configured for use in minimally-invasive medical intervention and diagnosis.

### BACKGROUND

Robotic instrument (e.g., catheter) systems and devices are well suited for use in performing minimally invasive medical procedures, as opposed to conventional techniques wherein the patient's body cavity is open to permit the surgeon's hands access to internal organs. For example, there is a need for a highly controllable yet minimally sized system to facilitate imaging, diagnosis, and treatment of tissues which may lie deep within a patient, and which may be accessed via naturally-occurring pathways such as blood vessels or the gastrointestinal tract, or small surgically-created pathways.

US Patent 5,762,458 discloses a system for performing minimally invasive cardiac procedures. The system comprises a pair of surgical instruments that are coupled to a pair of robotic arms which are coupled to a pair of master handles by a controller. The movement of the handles is scaled so that end effectors of the surgical instruments have a corresponding movement that is typically smaller than the movement performed by the hands of the surgeon.

### SUMMARY OF THE INVENTION

The robotic instrument system according to the invention includes a master input device and an instrument driver in communication with the controller; the instrument driver having a guide instrument interface including one or more guide instrument drive elements responsive to control signals generated, at least in part, by the master input device, for manipulating a guide instrument operatively coupled to the instrument interface. The master input device includes an operator interface coupled to a linkage assembly, with one or more load cells interposed between the operator interface and the linkage assembly, wherein control signals generated by the master input device are based at least in part on output signals generated by the one or more load cells in response to movement of the operator interface relative to the linkage assembly.

The operator interface is movable in at least three degrees of assembly. Preferably freedom relative to the linkage assembly. Preferably each of the one or more load cells is configured to detect localized tension or compression due to movement of the operator interface. In at least one embodiment, the operator interface is coupled to the link assembly by respective interface mounting members, between which the one or more load sensors are interposed. In one embodiment, the one or more load cells comprise three load cells and three adjacent springs interposed between the respective interface mounting members. In one embodiment, a shaft coupled to the operator interface passes through an arcuate slot formed in the interface mounting member and positioned between a pair of load cells that detect torsional loads applied to the operator interface. In one such embodiment, the operator interface comprises an operator interface mount coupled to the interface mounting member with a pivoting mounting fastener configured to allow the operator interface mount to rotate about the pivoting mounting fastener approximately in the plane of the interface mounting member, to thereby constrain movement of the shaft through the slot.

### BRIEF DESCRIPTION OF THE DRAWINGS

The drawings illustrate the design and utility of illustrated embodiments of the invention, in which similar elements are referred to by common reference numerals, and in which:
Fig. 1 illustrates a robotic catheter system in accordance with one embodiment;
Fig. 2 illustrates a robotic catheter system operator workstation in accordance with one embodiment;
Fig. 3 illustrates a perspective view of a master input device in accordance with one embodiment;
Fig. 4A illustrates a side view of a master input device in accordance with another embodiment;
Fig. 4B illustrates a magnified partial side view of the master input device embodiment of Fig. 4A;
Fig. 4C illustrates an exploded partial perspective view of the master input device embodiment of Fig. 4A;
Fig. 4D illustrates a partial perspective view of the master input device embodiment of Fig. 4A;
Fig. 4E illustrates a magnified partial rear view of the master input device embodiment of Fig. 4A;
Fig. 4F illustrates a magnified partial perspective view of the master input device embodiment of Fig. 4A;

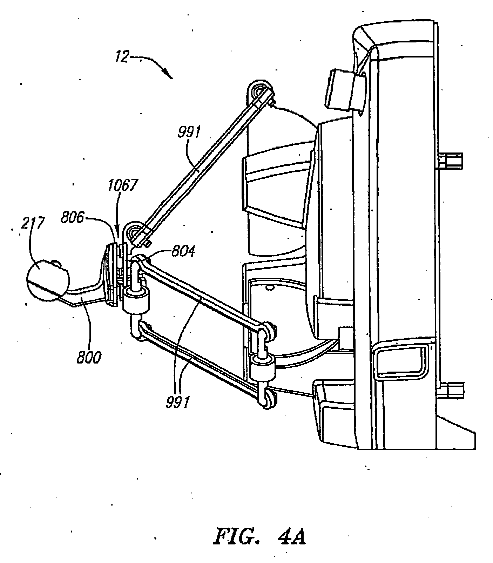

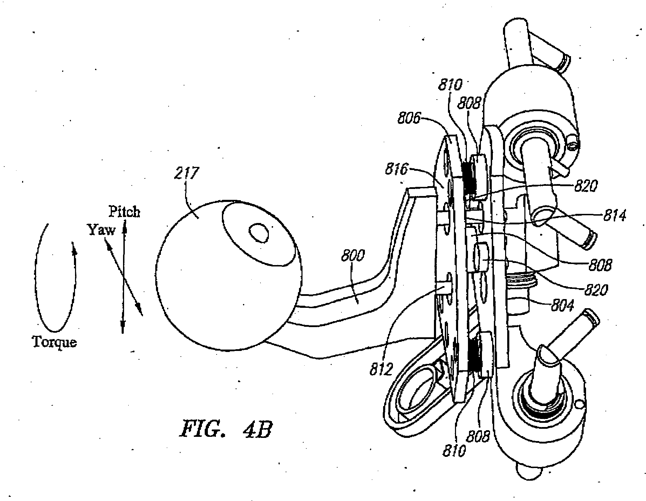

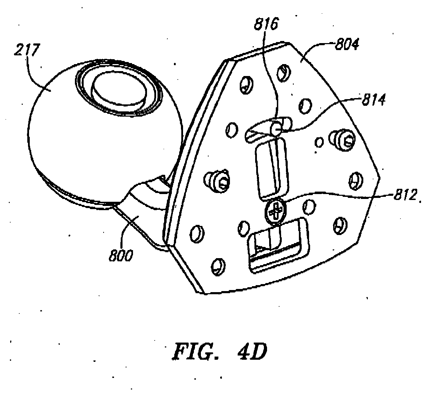

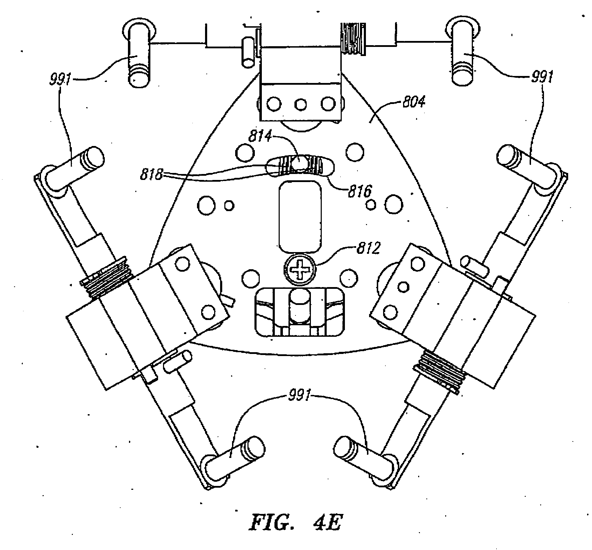

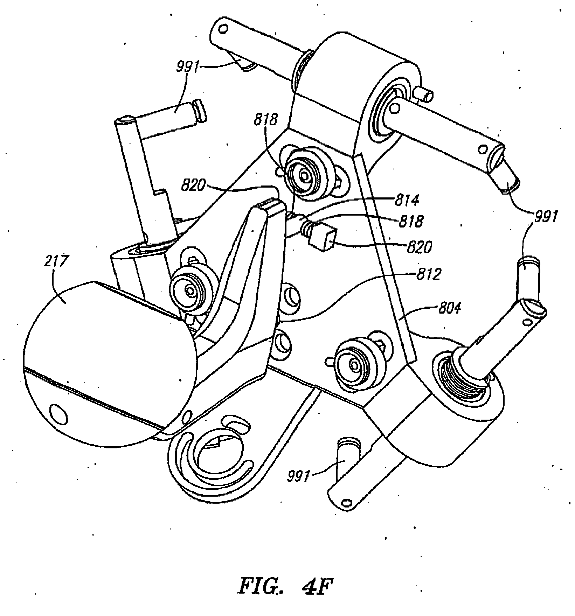

Fig. 4B illustrates a magnified partial side view of the master input device embodiment of Fig. 4A;

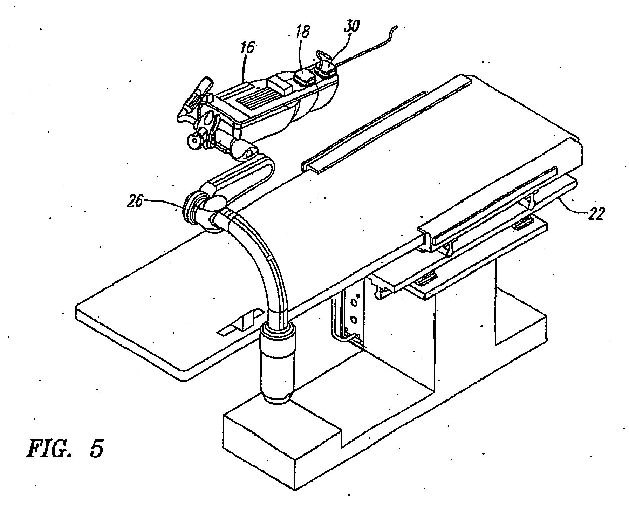

Fig. 5 illustrates a robotic catheter system.

### DETAILED DESCRIPTION OF THE ILLUSTRATED EMBODIMENTS

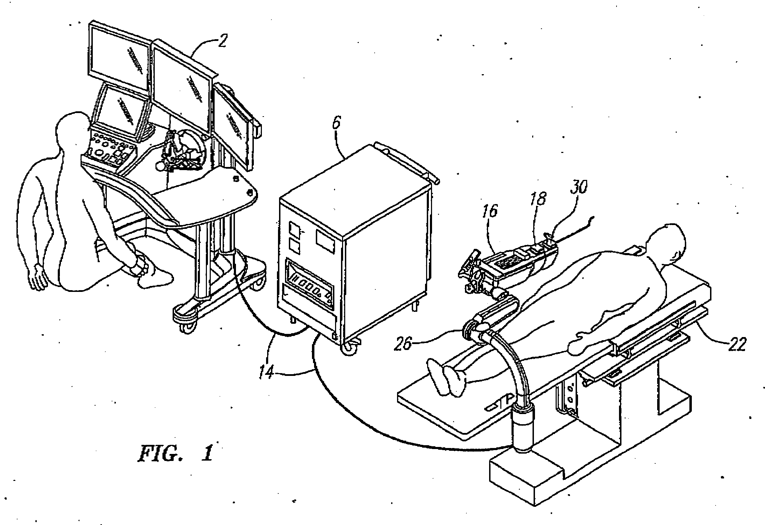

Referring to Figure 1, a robotic catheter system is depicted comprising an operator workstation (2), a movable computing and control system (6), a robotic instrument driver (16), a movable support assembly (26), which is removably mounted to an operating table (22), and an instrument set comprising a guide instrument (18) coaxially positioned through the working lumen of a sheath instrument (30), the guide instrument defining a working lumen configured to receive tools of various configurations (not shown in Figure 1) for interventional and diagnostic medical procedures.

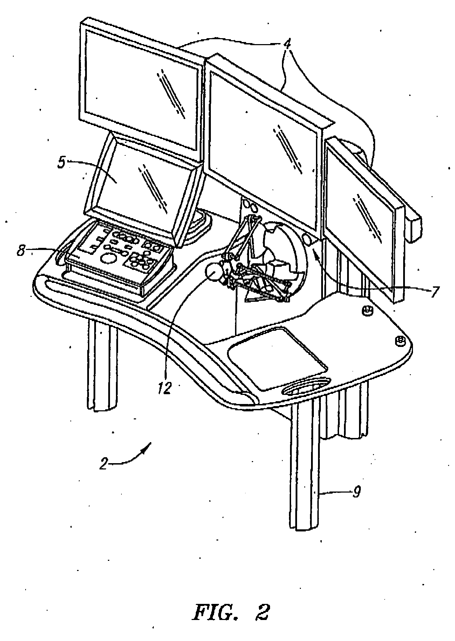

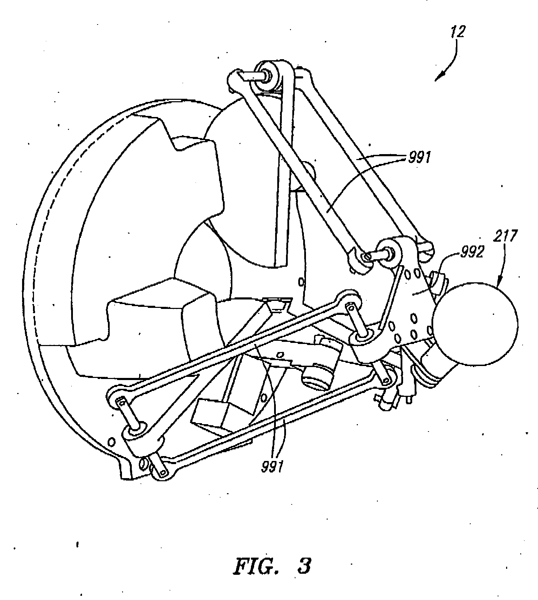

Referring to Figure 2, a closer view of an embodiment of an operator workstation (2) is depicted, comprising a master input device (12), an operator control console (8), a touchscreen interface (5), a device disabling switch (7), and several displays (4). Referring to Figure 3, a closer isometric view of one embodiment of a suitable master input device (12) is depicted having a substantially spherical operator interface (217) coupled to a master interface link assembly (991) by a master interface link assembly member (992) which is preferably fixedly attached to the operator interface (217). Suitable master input devices are available from manufacturers such as Sensible Devices Corporation under the trade name "Phantom™", or Force Dimension under the trade name "Omega™". Referring to Figures 4A-4F, another embodiment of a suitable master input device (12) is depicted.

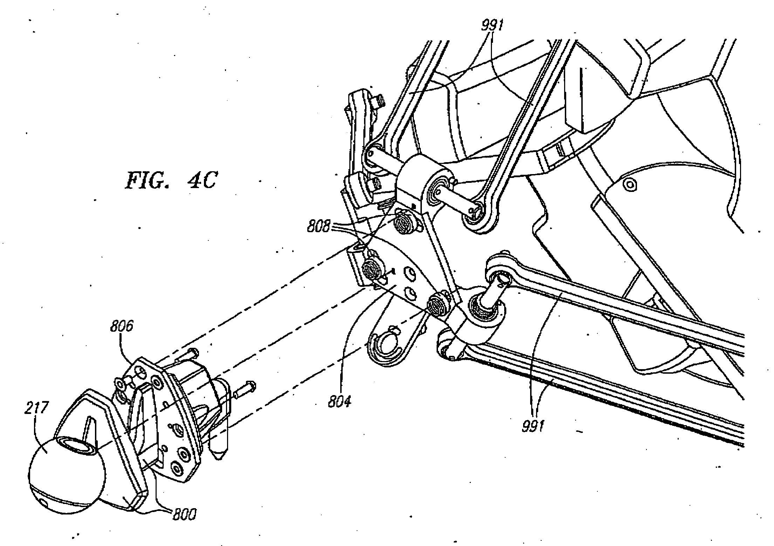

Referring to Figure 4A, a master input device (12) configuration is depicted having a three degree of freedom ("DOF") interface interposed between the operator interface (217) and the master input device link assembly member (804). Referring to Figure 4A, the operator interface (217) is coupled to the master interface link assembly (991) by two interface mounting members (804, 806), between which several load sensors are interfaced. Referring to Figure 4B, a close-up partial side view of the master input device is depicted to show three load cells (808) and three adjacent springs (810) interfaced between the master input device link assembly member (804) and the operator interface mounting member (806). Also depicted is a shaft (814) which is coupled to the operator interface mount (800), passed through an arcuate slot (816) formed in the operator interface mounting member (806), and positioned between two smaller load cells (820). The operator interface mount (800) is coupled to the operator interface mounting member (806) with a pivoting mounting fastener (812) which is configured to allow the operator interface mount (800) to rotate about the pivoting mounting fastener (812) approximately in the plane of the operator interface mounting member (806) to cause the shaft (814) to move through the slot (816) and cause the small load cells (820) to sense torque upon the operator interface (217), as illustrated in Figure 4B. The load cells (808) sensing forces between the operator interface mounting member (806) and the master input device link assembly member (804) may be utilized in concert to sense pitch and yaw loads at the operator interface (217). Thus, three degrees of freedom may be sensed with this variation of the master input device. In one embodiment, the torque degree of freedom may be interpreted as an attempt by the operator to actuate an electromechanical roll an instrument driver and associated elongate instruments. The pitch and yaw degrees of freedom may be assigned to various variables or degrees of freedom of the pertinent instrument driver and/or instrument. For example, in one embodiment, the pitch degree of freedom may be assigned to a sheath insertion actuation degree of freedom such that a positive pitch (beyond a selected minimum threshold load to prevent the pitch DOF from being accidentally triggered as the operator interface 217 is being gently moved about in 3-D space to steer the tip of the guide instrument, for example) may be interpreted as a signal that the operator wishes the sheath instrument to insert distally toward the distal tip of the guide instrument; similarly negative pitch can be assigned to negative insert - to pull the sheath instrument proximally. Figures 4C-4F show additional partial views ofthis variation of the master input device (12) to more fully illustrate the relative positions of the operator interface mount (800), master input device frameset (802), master input device link assembly member (804), operator interface mount member (806), load cells (808, 820), springs (810, 818), pivot mount interface (812), shaft (814), and arcuate slot (816) formed in the operator interface mounting member (806).

Referring to Figure 5, an isometric view of one embodiment of an instrument driver (16) coupled to an operating table (22) by a movable support assembly (26) is depicted. Guide (18) and sheath (30) instruments are depicted removably coupled to the instrument driver, as they preferably would be during a medical procedure utilizing the subject robotic catheter system.

While multiple embodiments and variations of the many aspects of the invention have been disclosed and described herein, such disclosure is provided for purposes of illustration only. Many combinations and permutations of the disclosed system are useful in minimally invasive surgery, and the system is configured to be flexible.

## Claims

1. A robotic instrument system, comprising:
a controller (6) including a master input device (12); and
an instrument driver (16) in communication with the controller (6), the instrument driver (16) having a guide instrument interface including one or more guide instrument drive elements responsive to control signals generated, at least in part, by the master input device (12), for manipulating a guide instrument (18) operatively coupled to the instrument interface,
the master input device (12) comprising an operator interface (217) coupled to a linkage assembly (991), with one or more load cells (808, 820) interposed between the operator interface (217) and the linkage assembly (991), wherein control signals generated by the master input device (12) are based at least in part on output signals generated by the one or more load cells (808, 820) in response to movement of the operator interface (217) relative to the linkage assembly (991),
**characterized in that** the operator interface (217) is movable in at least three degrees of freedom relative to the linkage assembly (991).

2. The system of claim 1, wherein each of the one or more load cells (808, 820) is configured to detect localized tension or compression due to movement of the operator interface (217).

3. The system of claims 1 or 2, wherein the operator interface (217) is coupled to the link assembly (991) by respective interface mounting members (804, 806), between which the one or more load sensors are interposed.

4. The system of claim 3, wherein the one or more load cells comprise three load cells (808) and three adjacent springs (810) interposed between the respective interface mounting members (804, 806).

5. The system of claims 3 or 4, further comprising a shaft (814) coupled to the operator interface, wherein the shaft (814) passes through an arcuate slot (816) formed in the interface mounting member (806), and is positioned between a pair of load cells (820) that detect torsional loads applied to the operator interface.

6. The system of claim 5, wherein the operator interface comprises an operator interface mount (800) coupled to the interface mounting member (806) with a pivoting mounting fastener (812) configured to allow the operator interface mount (800) to rotate about the pivoting mounting fastener (812) approximately in the plane of the interface mounting member (806), to thereby constrain movement of the shaft (814) through the slot (816).

## Patentansprüche

1. Eine Roboterinstrumentenvorrichtung, welche Folgendes umfasst:
ein Steuergerät (6), welches eine Haupteingabeeinrichtung (12) beinhaltet; und
einen Instrumentenantrieb (16), welcher mit dem Steuergerät (6) kommuniziert, wobei der Instrumentenantrieb (16) eine Führungsinstrumentenschnittstelle aufweist, welche ein oder mehrere Führungsinstrument-Antriebselemente beinhaltet, die auf zumindest teilweise durch die Haupteingabeeinrichtung (12) erzeugte Steuersignale reagiert, zur Manipulation eines Führungsinstruments (18), welche operativ mit der Instrumentenschnittstelle verbunden ist,
wobei die Haupteingabeeinrichtung (12) eine Bedienerschnittstelle (217) umfasst, die mit einer Verbindungsanordnung (991) verbunden ist, mit einer oder mehreren Kraftmesszellen (808, 820), die zwischen der Bedienerschnittstelle (217) und der Verbindungsanordnung (991) eingefügt sind, wobei Steuersignale, die durch die Haupteingabeeinrichtung (12) erzeugt werden, zumindest teilweise auf Ausgangssignale der einen oder der mehreren Kraftmesszellen (808, 820) in Antwort auf die Bewegung der Bedienerschnittstelle (217) relativ zu der Verbindungsanordnung (991) basieren,
**dadurch gekennzeichnet, dass** die Bedienerschnittstelle (217) in mindestens drei Freiheitsgraden relativ zu der Verbindungsanordnung (991) bewegt werden kann.

2. Die Vorrichtung nach Anspruch 1, wobei jede der einen oder mehreren Kraftmesszellen (808, 820) zur Detektion einer lokalen Spannung oder Kompression durch eine Bewegung der Bedienerschnittstelle (217) ausgebildet ist.

3. Die Vorrichtung nach Anspruch 1 oder 2, wobei die Bedienerschnittstelle (217) mit der Verbindungsanordnung (991) durch entsprechende Schnittstellenbefestigungsteile (804, 806) verbunden ist, zwischen denen die eine oder die mehreren Kraftmesszellen eingefügt sind.

4. Die Vorrichtung nach Anspruch 3, wobei die eine oder die mehreren Kraftmesszellen drei Kraftmesszellen (808) umfassen sowie drei angrenzende Federn (810), die zwischen den entsprechenden Schnittstellenbefestigungsteilen (804, 806) eingefügt sind.

5. Die Vorrichtung nach Anspruch 3 oder 4, welche weiterhin einen Schaft (814) umfasst, der mit der Bedienerschnittstelle verbunden ist, wobei sich der Schaft (814) durch einen gebogenen Schlitz (816) erstreckt, welcher in dem Schnittstellenbefestigungsteil (806) gebildet ist, und der zwischen einem Paar von Kraftmesszellen (820) angeordnet ist, welche Torsionskräfte detektieren, die auf die Bedienerschnittstelle ausgeübt werden.

6. Die Vorrichtung nach Anspruch 5, wobei die Bedienerschnittstelle einen Bedienerschnittstellenhalter (800) umfasst, der mit dem Schnittstellenbefestigungsteil (806) mit einer schwenkbaren Halterung (812) verbunden ist, die derart ausgebildet ist, um den Bedienerschnittstellenhalter (800) um die schwenkbare Halterung (812) näherungsweise in der Ebene des Schnittstellenbefestigungsteils (806) rotieren zu lassen, um so die Bewegung des Schafts (814) durch den Schlitz (816) einzuschränken.

## Revendications

1. Système d'instrument robotisé, comprenant :
un contrôleur (6) incluant un maître-dispositif d'entrée (12), et
un dispositif de commande d'instrument (16) en communication avec le contrôleur (6), le dispositif de commande d'instrument (16) ayant une interface d'instrument de guidage incluant un ou plusieurs éléments de commande d'instrument de guidage répondant à des signaux de contrôle générés, au moins partiellement, par le maître-dispositif d'entrée (12), pour manipuler un instrument de guidage (18) couplé de manière opérationnelle à l'interface d'instrument,
la maître-dispositif d'entrée (12) comprenant une interface opérateur (217) couplée à un assemblage à liaisons (991), avec une ou plusieurs cellules de charge (808, 820) interposées entre l'interface opérateur (217) et
l'assemblage à liaisons (991), où les signaux de contrôle générés par le maître-dispositif d'entrée (12) sont basés au moins partiellement sur des signaux de sortie générés par la ou les cellules de charge (808, 820) en réponse au mouvement de l'interface opérateur (217) par rapport à l'assemblage à liaisons (991),
**caractérisé en ce que** l'interface opérateur (217) est mobile selon au moins trois degrés de liberté par rapport à l'assemblage à liaison (991).

2. Le système de la revendication 1, où la ou chacune des cellules de charge (808, 820) est configurée pour détecter une tension ou compression localisée due au mouvement de l'interface opérateur (217).

3. Le système des revendications 1 ou 2, où l'interface opérateur (217) est couplée à l'assemblage à liaisons (991) par des membres respectifs (804, 806) de montage de l'interface, entre lesquels la ou les capteurs de charge sont interposés.

4. Le système de la revendication 3, où la ou les cellules de charge comprennent trois cellules de charge (808) et trois ressorts adjacents (810) interposés entre les membres respectifs (804, 806) de montage de l'interface.

5. Le système de la revendication 3 ou 4, comprenant en outre un axe (814) couplé à l'interface opérateur, où l'axe (814) passe au travers d'une rainure courbée (816) formée dans le membre (806) de montage de l'interface, et est positionnée entre une paire de cellules de charge (820) qui détectent les charges de torsion appliquées à l'interface opérateur.

6. Le système de la revendication 5, où l'interface opérateur comprend un support (800) d'interface opérateur couplé au membre de montage de l'interface (806) avec une fixation de montage pivotante (812) configurée pour permettre au support d'interface opérateur (800) de tourner autour de la fixation de montage pivotante (812) approximativement dans le plan du membre de montage de l'interface (806), pour afin de contraindre le mouvement de l'axe (814) au travers de la rainure (816).
